# EUROPEAN PATENT SPECIFICATION

(11) **EP 0 432 841 B1**
(45) Date of publication and mention of the grant of the patent: **27.03.1996**
(21) Application number: 90203216.8
(22) Date of filing: 06.12.1990
(51) Int. Cl.: C07D 498/10, G03C 1/685, C09K 9/02

(54) **Spiro-indoline-oxazine compounds with photochromatic and photosensitizing characteristics and the process for their preparation**
Photochrome und photoempfindliche Spiroindolinoxazinverbindungen und Verfahren zu ihrer Herstellung
Composés photochromiques et photosensibles de spiroindolineoxazine et procédé pour leur préparation

(30) Priority: 12.12.1989 IT 2266089
(43) Date of publication of application: 19.06.1991
(73) Proprietor: GREAT LAKES CHEMICAL ITALIA S.r.l., I-20138 Milano (IT)
(72) Inventor: Castaldi, Graziano, I-28072 Briona, Novara (IT); Allegrini, Pietro, I-20097 San Donato Milanese, Milan (IT); Crisci, Luciana, I-20079 Sant'Angelo Lodigiano, Milan (IT); Renzi, Fiorenzo, I-20064 Gorgonzola, Milan (IT)
(74) Representative: Fusina, Gerolamo

(56) References cited:
- EP-A- 0 146 135
- EP-A- 0 245 020
- EP-A- 0 277 639
- US-A- 4 785 097
- CHEMICAL ABSTRACTS, vol. 113, no. 12, 17 September 1990, Columbus, Ohio, US; abstract no. 106503H, T. OKUDAIRA: 'Bis(spiro(indolinonaphthoxazine)) compounds as photochromic materials with high heat resistance' page 639 ; & JP-A- 02080490, 20 March 1990

## Description

This invention relates to new photochromatic compounds and the photochromatic articles which contain them. The invention also relates to the process for preparing these compounds and their use as photosensitizing agents.

Photochromatic compounds are substances having the characteristic of reversibly changing their colour and/or degree of light transmission when exposed to certain types of electromagnetic radiation and sunlight, to return to their initial colour and light transmission state when the light source is removed.

There are many known substances possessing photochromatic characteristics and pertain to various classes of organic and inorganic compounds, as described for example in "Photochromism", G.H. Brown (Ed.), vol. III, of the Weissberger series "Techniques of Organic Chemistry", Wiley Interscience, New York (1971).

The most well known photochromatic organic compounds pertain to the spiro-indoline-oxazine class and are able to confer photochromatic characteristics on polymerized organic materials used in solar filters, optical articles, optical memories, printing, photography, fabrics, decorative articles and toys etc., as described for example in U.S. patents 3,562,172, 3,578,602, 4,215,010 and 4,342,668, and in European patent applications publication Nos. 134,633 and 141,407.

Compared with other known organic photochromatic compounds (such as those of the spiro-pyran class), known photochromatic compounds of the spiro-indoline-oxazine class have the advantage of a much higher fatigue resistance when subjected to repeated coloration and decoloration cycles and a much higher resistance to ageing by exposure to sunlight or in artificial ageing tests.

However, the organic photochromatic compounds of the known art are practically colourless in their deactivated state, whether in solution in common organic solvents or incorporated into transparent polymer materials, and generally become blue when activated. This blue colour is a drawback in its use in photochromatic optical articles, especially in the ophthalmic sector, in which more neutral colours such as grey are preferred.

In addition the photochromatic effect obtained is in many cases of little use with regard to transmittance variation in the visible spectrum. In other cases this variation, although satisfactory at low temperatures, is depressed to unacceptably low values as the temperature increases, even if this latter is maintained within the range of values specified for practical use. Finally, spiro-indoline-oxazine activation effected under controlled laboratory conditions by irradiation with ultraviolet light of different wavelengths from about 320 to about 380 nm is often not obtained with the same satisfactory speed and intensity when effected by exposure to the frequency spectrum and intensity of sunlight, as required by most uses. Sometimes, by using in association with the photochromatic compound a photosensitizer, as described in "Photochromism", G.H. Brown (Ed.), vol. III, of the Weissberger series "Techniques of Organic Chemistry", Wiley Interscience, New York (1971) and in French patent 2,618,812, greater activation speeds and coloration intensities can be obtained, but without obviating the problems of stability and duration of the photochromatic effect. Examples of such photosensitizers are aromatic compounds (benzene, naphthalene, anthracene) or aliphatic or aromatic ketones (acetone, benzophenone, anthraquinone, xanthone). However in many cases a molecule which acts as a photosensitizer for a photochromatic compound under determined experimental conditions can act as a photoinhibitor if the conditions and/or the photochromatic substrate vary , as reported for example in Journal of the American Chemical Society (1964) 86, page 5687.

Other photochromatic compounds are disclosed by EP-A-0146135; it teaches that, in order to obtain good photochromatic characteristics, it is necessary to have steric hindrance on the spiro-carbon. In particular, it teaches that compounds having a hydrogen atom in 2' position and three methyl groups in 1 and 3 positions (corresponding to R⁷ and R⁴, R⁵ and R⁶ substituents in the compounds of formula (I) of EP'135) are not endowed with good photochromatic characteristics.

EP-A-0245020 discloses plastic organic photochromatic articles having a photochromic compound incorporated therein or applied thereto, said photochromatic compound necessarily presenting an amino group on the nucleus of the benzene ring of the oxazine substituent.

The object of the present invention is to overcome the aforesaid drawbacks of the known art by providing new photochromatic compounds of the spiro-indoline-oxazine class.

In accordance therewith a first aspect of the present invention is the provision of new photochromatic and photosensitizing compounds of the spiro-indoline-oxazine class definable by the general formula (I): 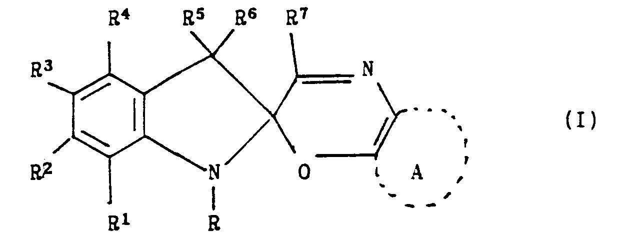
where:
- R represents a methyl group;
- R¹ to R⁴, which can be identical or different, each independently represent a hydrogen atom; a linear or branched C₁-C₅ alkyl group; a said C₁-C₅ alkyl group substituted with 1 to 5 atoms of halogen chosen from fluorine, chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; an amino group; a C₁-C₅ monoxyalkyl amino group; a C₁-C₅ dialkyl amino group; a C₃-C₁₀ cycloalkyl amino group; a piperidino, piperazino or morpholino group; a carboxyl group; a C₁-C₅ carboxyalkyl group; a C₂-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluene-sulphonic and p-chlorotoluenesulphonic groups; or an aryl group chosen from phenyl, diphenyl and naphthyl groups;
- R⁵ and R⁶, both represent a methyl group;
- R⁷ represents a hydrogen atom;
- A represents a monocyclic or polycyclic arene group containing at least one C=O carbonyl function on the nucleus or in the side chain, and chosen from those representable by the following formulas (II), (III), (IV), (V) or (VI): 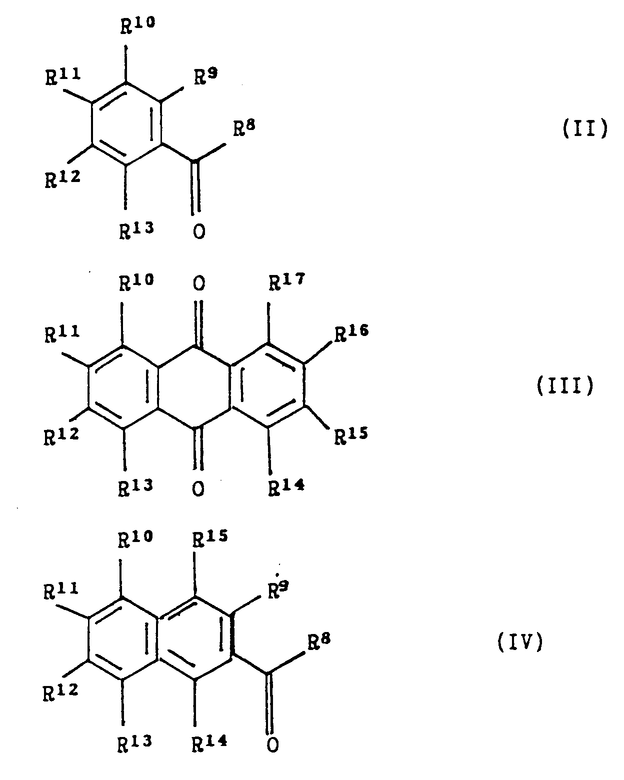 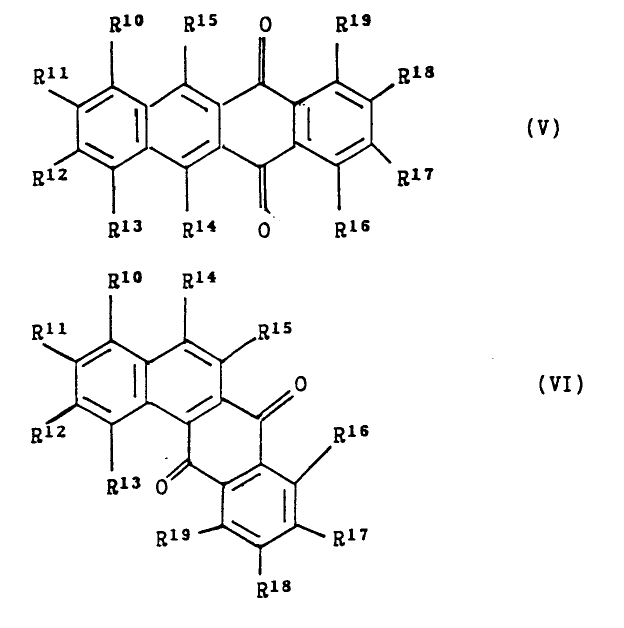 where:
- R⁸ represents a hydrogen atom; a halogen atom chosen from chlorine and bromine; a linear or branched C₁-C₅ alkyl group; a C₂-C₅ alkenyl group; a monocyclic or polycyclic aryl group; or a heteroaryl group chosen from phenyl, naphthyl, anthracyl, furanyl, thiophenyl, quinolyl and pyrrolyl; or a said aryl or heteroaryl group substituted with 1 to 4 groups chosen from a halogen atom selected from fluorine, chlorine and bromine, linear or branched C₁-C₅ alkyl groups, hydroxy groups, C₁-C₅ alkoxy groups, nitro groups, cyano groups, amino groups, C₁-C₅ monoalkyl amino groups; C₁-C₅ dialkyl amino groups; piperidino groups, piperazino groups or morpholino groups;
- two adjacent of R¹⁰ to R¹³ represent the position of fusion with the oxazine ring in general formula (I), the others having the same meaning as R⁹ AND R¹⁴-R¹⁹;
- R⁹ and R¹⁴-R¹⁹ represent a hydrogen atom; a linear or branched C₁-C₅ alkyl group; a C₁-C₅ alkyl group substituted with 1 to 5 atoms of halogen chosen from fluorine, chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; a carboxyl group; a C₁-C₅ carboxyalkyl group; a C₁-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluenesulphonic and p-chlorotoluenesulphonic groups; or an aryl group chosen from phenyl, diphenyl and naphthyl groups.

In the preferred embodiment, in formula (I):
- R represents a methyl group;
- R¹-R⁴, which are identical or different, each independently represent a hydrogen atom, a fluorine, chlorine or bromine atom, or a methyl, isopropyl, trifluoromethyl, hydroxymethyl, benzyl, hydroxy, methoxy, amino, piperidino, morpholino, carboxyl, carboxymethyl, N,N-dimethylcarboxyamide, cyano, nitro or phenyl group;
- R⁵ and R⁶, both represent a methyl group;
- R⁷ represents a hydrogen atom;
- A is one of the groups of formulas (II) to (VI) in which:
- R⁸ represents a hydrogen atom, or a methyl, isopropyl, phenyl, p-N,N-dimethyl aminophenyl, p-cyano phenyl, p-nitro phenyl, p-methoxy phenyl, naphthyl, 2-thiophenyl, 2-furanyl or 4-pyridyl group;
- two adjacent of R¹⁰-R¹³ represent the position of fusion with the oxazine ring in general Formula (I), the others then representing each independently a hydrogen atom, a fluorine, chlorine or bromine atom, or a methyl, isopropyl, trifluoromethyl, hydroxymethyl, benzyl, hydroxy, carboxyl, carboxymethyl, N,N-dimethylcarboxyamide, cyano, nitro or phenyl group;
- R⁹ and R¹⁴-R¹⁹ each independently represent a hydrogen atom, a fluorine, chlorine or bromine atom, or a methyl, isopropyl, trifluoromethyl, hydroxymethyl, benzyl, hydroxy, methoxy, carboxyl, carboxymethyl, N,N-dimethylcarboxyamide, cyano, nitro or phenyl group.

Specific examples of preferred photochromatic compounds according to the present invention are:
- 1,3,3-trimethyl-8'-oxophenyl-spiro-indoline-2,3'-[3H]naphtho(2,1-b)-1,4-oxazine (IA);
- 1,1,1-trimethyl-spiro-indoline-2,3'-[3H]benzo[a]anthracene-7',12'-dione(3,4-b)-1,4-oxazine (IB); and.

The compounds (I) according to the present invention can be prepared by reacting a nitroso derivative of the compounds of formula (II), (III), (IV), (V) or (VI), in which two adjacent of R¹⁰-R¹³ represent one a -N=O group an the other a -OR²⁰ group where R²⁰ is a hydrogen atom, a metal cation chosen from sodium, potassium, lithium and copper cations; an ammonium or C₁-C₅ alkylammonium cation; a C₁-C₅ alkylcarbonyl group; a C₁-C₅ alkylsulphonyl group or arylsulphonyl group;
with a compound of formula (VII): 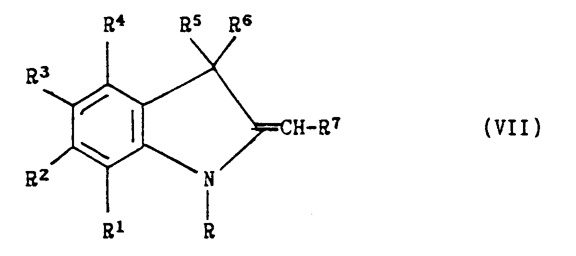
where the substituents R and R¹-R⁷ have the aforesaid meaning.

The nitroso derivatives of the compounds (II), (III), (IV), (V) and (VI) can be prepared by known methods, such as those described in Organic Synthesis Collective Volume 3, page 411, or in U.S. patent 3,285,972.

The compounds (VII) can be prepared by known methods. as described for example in Journal American Chemical Society (1941), 63, page 2024; or in Bull. Soc. Chim. Franc. (1968) page 2066.

The reaction is generally conducted by adding the compound (VII) to a solution or suspension of the nitroso derivative of one of the compounds (II), (III), (IV), (V) and (VI), in an inert organic solvent, possibly in the presence of a tertiary amine, operating at a temperature of between 0 and 150°C and preferably between 0 and 80°C, for a time of between about 1 minute and 24 hours.

The inert solvents used in the reaction can be chosen from aliphatic or aromatic hydrocarbons (such as pentane, hexane, heptane, benzene, toluene and xylene); chlorinated aliphatic or aromatic hydrocarbons (such as dichloromethane, 1,2-dichloroethane and chlorobenzene); aliphatic or aromatic ethers (such as diethyl ether, tetrahydrofuran and diphenylether); alcohols (such as methanol, ethanol, isopropanol and n-butanol); esters (such as ethyl acetate); amides (such as dimethylformamide); nitriles (such as acetonitrile); carbonates (such as dimethylcarbonate); and water.

In the reaction the nitroso derivatives of the compounds (II), (III), (IV), (V) or (VI) can be used in quantities varying from 0.1 to 10 moles per mole of the compound (VII), but preferably equimolecular or approximately equimolecular quantities are used.

If the reaction is conducted in the presence of a tertiary amine this can be used in a quantity of between 0.1 and 2 moles per mole of the nitroso derivative, but equimolecular or approximately equimolecular quantities are preferably used. Examples of tertiary amines suitable for the purpose are: triethylamine, pyridine, 4-N,N-dimethylaminopyridine, N-methylpiperidine, and N-methylmorpholine.

On termination of the reaction the compounds (I) are isolated by normal methods, for example by evaporating the solvent under vacuum, and then purified for example by crystallization or chromatography. Solvents suitable for crystallization include pentane, hexane, heptane, toluene, ethyl ether, methanol, ethanol, isopropanol, n-butanol, tetrahydrofuran, acetone, methylethylketone, ethyl acetate, dimethylcarbonate, acetonitrile and relative mixtures.

The compounds (I) according to the present invention are crystalline products which are colourless or for example yellow, orange or red in colour. Their solutions in the common organic solvents, when not exposed to light sources, are colourless or yellow or yellow-orange in colour. When these solutions are exposed to a light source (either visible or ultraviolet) they rapidly assume an intense yellow, yellow-orange, red, blue or green coloration. The coloration falls off rapidly when the light source is removed. The intensity of the coloration observed for solutions of the compounds (I) is much higher, for equal concentrations and experimental conditions, than that of known photochromatic compounds of the spiro-indoline-oxazine class as can be seen from the graphs of Figure 1.

Specifically, the graphs 1, 2 and 3 of Figure 1 represent the transmittance curves of 10-³M toluene solutions of the deactivated form (ie before exposure to the light source) and of the activated form (ie after exposure to the light source) of a known photochromatic compound (VIII): 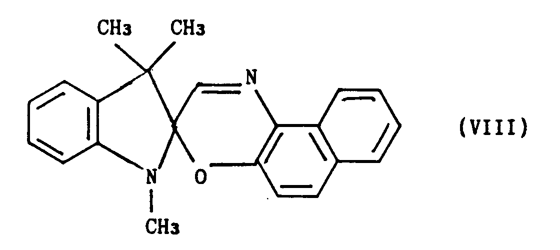
and of the photochromatic compounds (IA) and (IB) of the present invention, the determinations being made by a Macbeth spectrophotometer. Activation is effected by irradiation for 360 seconds with a high pressure mercury lamp.

Aliphatic and aromatic ketones are known photosensitizers and/or photoinhibitors, as reported for example in the text "Technologie Photochimique", A.M. Braun, M.T. Maurette, E. Oliveras, Press Polytechniques Romandes, Lausanne (1986). For example when toluene solutions of the known compound of spiro-indoline-oxazine structure (VIII) are exposed to a light source in the presence of an equimolecular quantity of benzophenone or anthraquinone, an inhibition effect on photochromatic activity is noted, as shown in graphs 1, 4 and 5 of Figure 2. Specifically, graph 4 of Figure 2 shows respectively the transmittance curves of the deactivated form and the form at maximum activation of the compound (VIII) and of the compound (VIII) with an equimolecular quantity of benzophenone. Graph 5 of Figure 2 shows respectively the transmittance curves of the deactivated form and the form at maximum activation of the compound (VIII) and of the compound (VIII) with an equimolecular quantity of anthraquinone. Surprisingly, the 10-³M toluene solutions of compounds (IA) and (IB) under the same experimental conditions (Figure 1) show high activation kinetics and a high coloration (transmittance variation), in contrast to those of the known compound (VIII) in the presence of benzophenone and anthraquinone. In addition the compounds of the present invention act as photosensitizers for example for known photochromatic compounds. Graph 6 of Figure 3 shows respectively the transmittance curves for for 2.10-⁴M toluene solutions at maximum activation of the known compound (IX): 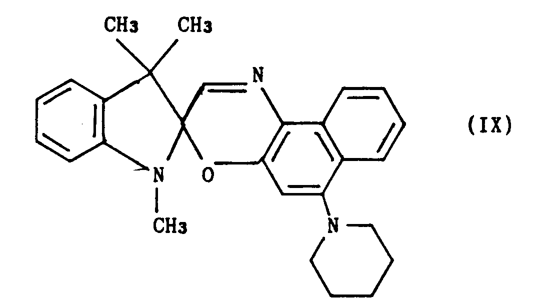
and of the compound (IB) of the present invention. Graph 7 of Figure 3 shows respectively the theoretical transmittance curve at maximum activation for a 1:1 mixture of the compounds (IX) and (IB) and the experimental curve at maximum activation for the same mixture. Surprisingly the experimental transmittance curve shows much lower transmittance values than the theoretical, and consequently a much higher photochromatic effect than expected.

The compounds (I) of the present invention demonstrate high photochromatic activity even on irradiation with visible light sources and hence with sunlight, with high activation and deactivation rates. Activation of the compounds (I) can in fact be obtained with a flash of visible light, such as that of photocopier machines, generally between 450 and 550 nm. This characteristic means that the compounds (I) of the present invention can be used as security markers for confidential documents. In addition the high activation/deactivation rate and stability of the compounds (I) enables transparent optical materials such as lenses, ophthalmic lenses, contact lenses, sunglasses, windows for vehicles and transport means in general, and windows in the building sector. The compounds (I) of the present invention are particularly useful for conferring photochromatic characteristics on articles of polymer materials used as solar filters, optical articles, optical memories, prints, photographs, fabrics, decorative articles and toys.

The compounds (I) can be applied to the surface or incorporated in the required articles, by suitable methods. Certain photochromatic polymers can be obtained by dispersing the photochromatic compound homogeneusly within the polymer mass and moulding (e.g. by injection, compression and the like). Alternatively the photochromatic compound can be dissolved in a suitable solvent together with a polymer (such as polymethyl methacrylate, polyvinyl alcohol, polyvinyl butyral, cellulose butyrate acetate, epoxy, polysiloxane or urethane resin etc.) and deposited on a transparent support to form a photochromatic coating after evaporating the solvent. Again, the photochromatic compound can be added to a polymerizable monomer such as methyl methacrylate, so that after polymerization conducted in the presence of a suitable initiator such as azobisisobutyronitrile, they remain uniformly incorporated in the formed resin. According to a further method, the photochromatic compound can be dissolved in a suitable solvent in the presence of a resin, as heretofore described, this solution then being used to form a photochromatic film or sheet containing the uniformly dispersed photochromatic compound, by evaporating the solvent. Finally, the photochromatic compound can be applied to a transparent substrate (such as polycarbonate, polymethyl methacrylate, or polydiethyleneglycol bis(allyl carbonate)) by surface impregnation, effected by bringing the substrate into contact at a suitable temperature with a solution or dispersion containing the photochromatic compound.

The following experimental examples are provided to better illustrate the present invention.

### EXAMPLE 1

### Preparation of 3-hydroxy-4-nitroso-benzo[a]anthracene-7,12-dione

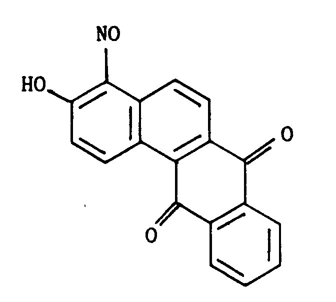

A solution of sodium hydroxide (0.72 g) in water (0.72 g) is added under stirring at ambient temperature to a suspension of 3-hydroxy-benzo[a]anthracene-7,12-dione, prepared as described in Journal Organic Chemistry (1986) 51 page 3502, (5 g; 18 mmoles) in butanol (25 ml). N-butylnitrite (2 g; 19.4 mmoles) is added over a period of 1 hour to the mixture, which is maintained at 60°C.

The reaction mixture is maintained at 60°C for 4 hours, cooled to ambient temperature and filtered. The solid is washed with toluene (2 x 10 ml), dried under vacuum and suspended in water (25 ml). Acetic acid (2 ml) is added to the suspension over a period of 30 minutes, operating at ambient temperature under stirring.

The suspension is kept stirred at ambient temperature for a further hour and then filtered. The solid is washed with water (3 x 20 ml) and dried under vacuum. In this manner 4.5 g of the required compound of the title are obtained, with sufficient purity for use in the next reaction.

### EXAMPLE 2

### Preparation of 1,3,3-trimethyl-spiro-indoline-2,3'-[3H]-benzo[a] anthracene-7',12'-dione(3,4-b)-1,4-oxazine (IB)

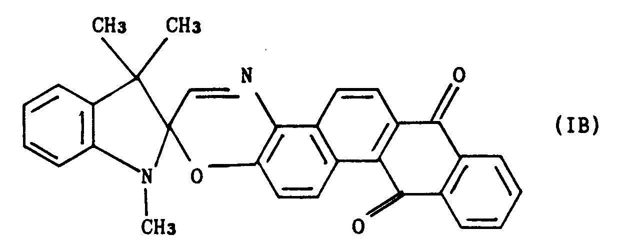

1,3,3-trimethyl-2-methylene-indoline (2.08 g; 12 mmoles) is added under stirring at ambient temperature to a suspension of 3-hydroxy-4-nitroso -benzo[a]anthracene-7,12-dione (4.4 g; 14.5 mmoles) in toluene (60 ml). The mixture is heated to 70°C for 3 hours, then cooled to ambient temperature, diluted with toluene (60 ml) and washed with 3% aqueous hydrochloric acid, and finally dried with sodium sulphate. After evaporating the solvent under vacuum a crude product is obtained which on silica gel chromatography (eluent toluene) provides 1 g of the required product of the title. Melting point 234.5°C (DSC).
¹H-NMR (200 MHz, CDCl₃-TMS) δ (ppm): 1.35 (3H, s), 1.36 (3H, s), 2.77 (3H, s, NCH₃), 6.6-9.6 (13H, m).
Mass (m/e): 458.

| Elemental analysis: | | | |
|---|---|---|---|
| Calculated: | C 78.6%; | H 4.8%; | N 6.1% |
| Found: | C 78.1%; | H 4.7%; | N 5.9% |

### EXAMPLE 3

### Preparation of 6-hydroxy-5'-nitroso-2'-naphthyl phenyl ketone

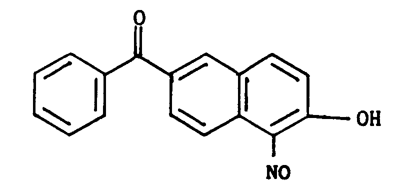

A solution of sodium hydroxide (0.16 g) in water (0.16 g) is added under stirring at ambient temperature to a suspension of 6-hydroxy-2'-naphthyl phenyl ketone, prepared as described in Journal of Am. Chem. Soc. (1950) 72 page 3215, (1 g; 4 mmoles) in n-butanol ( 5 ml). N-butylnitrite (0.42 g; 4 mmoles) is added over a period of 1 hour to the mixture, which is maintained at 60°C. The reaction mixture is maintained at 60°C for 4 hours, cooled to 0°C and filtered. The solid is washed with ethyl ether, dried under vacuum and suspended in water (5 ml). Acetic acid (0.5 ml ) is added to the suspension over a period of 30 minutes, operating at ambient temperature under stirring. The suspension is kept stirred at ambient temperature for a further hour and then filtered. The solid is washed with water and dried under vacuum. In this manner 0.8 g of the required compound of the title are obtained, with sufficient purity for use in the next reaction.

### EXAMPLE 4

### Preparation of 1,3,3-trimethyl-8'-oxophenyl-spiro-indoline-2,3'-[3H]-naphtho(2,1-b)-1,4-oxazine (IA)

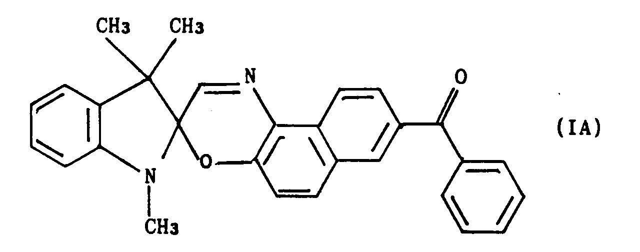

1,3,3-trimethyl-2-methylene-indoline (1.06 g; 6.1 mmoles) is added under stirring at ambient temperature to a suspension of 6'-hydroxy-4-nitroso-2'-naphthyl phenyl ketone (1.7 g; 6.1 mmoles) in toluene (30 ml). The mixture is heated to 70°C for 6 hours, then cooled to ambient temperature, diluted with toluene (30 ml) and washed with 3% aqueous hydrochloric acid, and finally dried with sodium sulphate. After evaporating the solvent under vacuum a crude product is obtained which on silica gel chromatography eluent hexane:ethyl acetate, 5:1 by volume) provides 0.9 g of the required product of the title. Melting point 180°C (DSC).
¹H-NMR (200 MHz, CDCl₃-TMS) δ (ppm): 1.34 (3H, s), 1.36 (3H, s), 2.76 (3H, s, NCH₃), 6.5-8.6 (15H, m).
Mass (m/e): 432.

| Elemental analysis: | | | |
|---|---|---|---|
| Calculated: | C 80.6%; | H 5.6%; | N 6.6% |
| Found: | C 79.9%; | H 5.6%; | N 6.3% |

### EXAMPLE 5

### Preparation of 1,3,3-trimethyl-8'-oxophenyl-6'-piperidinyl-spiro-indoline-2,3'-[3H]-naphtho(2,1-b)-1,4-oxazine (IC)

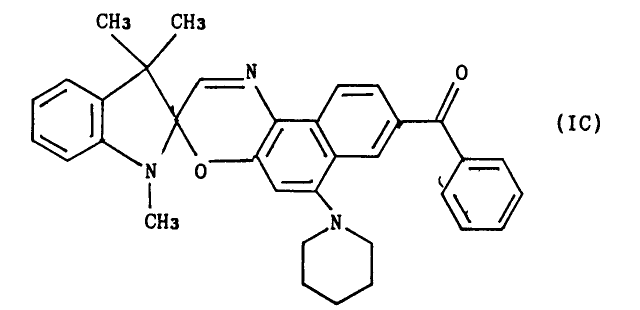

Piperidine (0.21 ml; 2.17 mmoles) is added under stirring at ambient temperature to a suspension of 6'-hydroxy-4-nitroso-phenyl ketone (0.6 g; 2.17 moles) in toluene (8 ml). The reaction mixture is heated to 70°C for 30 minutes. The resultant solution is added to a solution of 1,3,3-trimethyl-2-methylene-indoline (0.187 g; 1.08 mmoles) in toluene (2 ml) mantained at 70°C. The reaction mixture is maintained at 70°C for 3 hours, then cooled to ambient temperature, diluted with toluene (90 ml) and washed with 3% aqueous hydrochloric acid (100 ml), and finally dried with sodium sulphate. After evaporating the solvent under vacuum a crude product is obtained which on silica gel chromatography (eluent toluene) provides the required product of the title.
¹H-NMR (200 MHz, CDCl₃-TMS) δ (ppm): 1.35 (6H, s), 1.35 (2H, m), 1.58 (4H, m), 2.75 (3H, s, NCH₃), 3.02 (4H, m), 6.6-8.7 (14H, m).
Mass (m/e): 501.

| Elemental analysis: | | | |
|---|---|---|---|
| Calculated: | C 81.4%; | H 6.6%; | N 5.6% |
| Found: | C 81.0%; | H 6.7%; | N 5.4% |

## Claims (Claims for the following Contracting State(s): AT, BE, CH, DE, DK, FR, GB, GR, LI, LU, NL, SE)

1. Photochromatic and photosensitizing compounds of the spiro-indoline-oxazine class definable by the general formula (I): 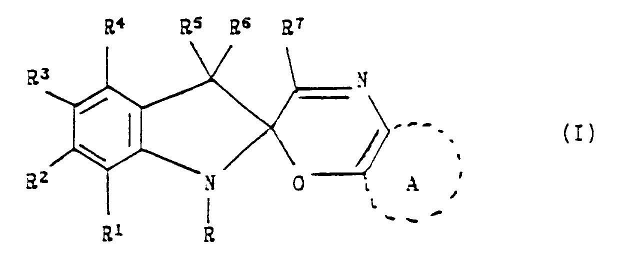 where:
- R represents a methyl group;
- R¹ to R⁴, which can be identical or different, each independently represent a hydrogen atom; a linear or branched C₁-C₅ alkyl group; a said C₁-C₅ alkyl group substituted with 1 to 5 atoms of halogen chosen from fluorine, chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; an amino group; a C₁-C₅ monoxyalkyl amino group; a C₁-C₅ dialkyl amino group; a C₃-C₁₀ cycloalkyl amino group; a piperidino, piperazino or morpholino group; a carboxyl group; a C₁-C₅ carboxyalkyl group; a C₂-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluenesulphonic and p-chlorotoluenesulphonic groups; or an aryl group chosen from phenyl, diphenyl and naphthyl groups;
- R⁵ and R⁶, both represent a methyl group;
- R⁷ represents a hydrogen atom;
- A represents a monocyclic or polycyclic arene group containing at least one carbonyl function (C=O) on the nucleus or in the side chain, and chosen from those representable by the following formulas (II), (III), (IV), (V) or (VI): 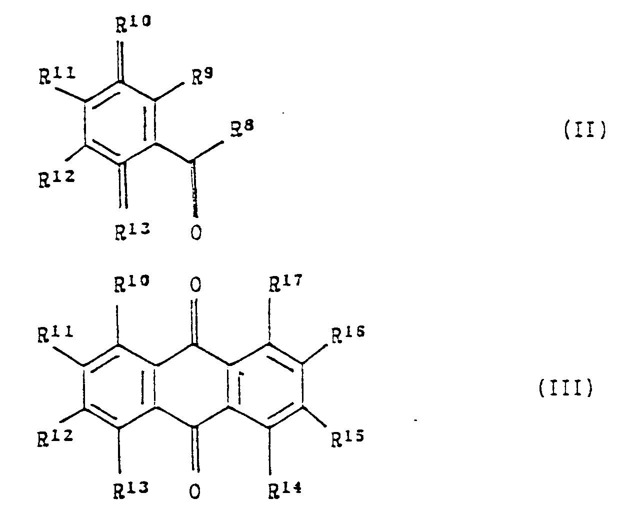 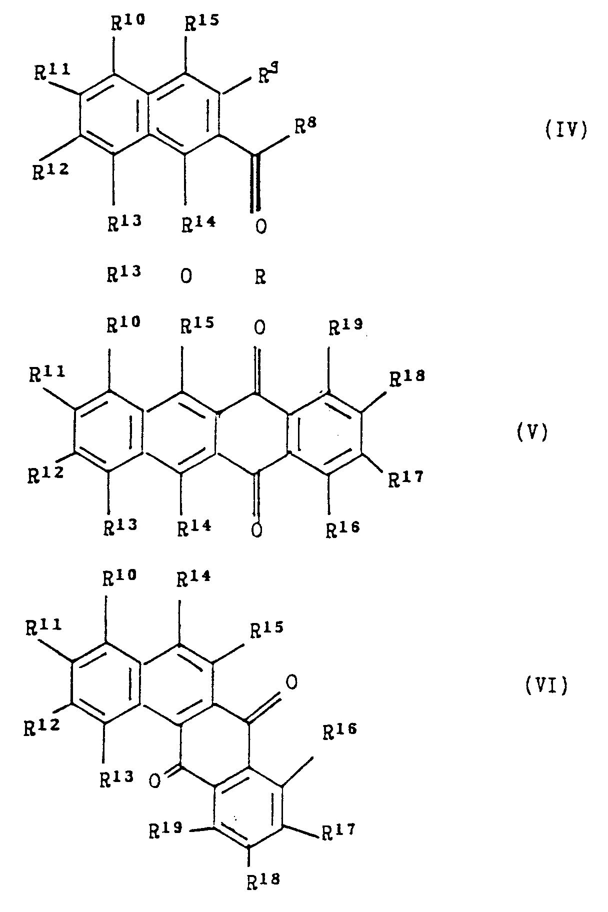 where:
- R⁸ represents a hydrogen atom; a halogen atom chosen from chlorine and bromine; a linear or branched C₁-C₅ alkyl group; a C₂-C₅ alkenyl group; a monocyclic or polycyclic aryl group; or a heteroaryl group chosen from phenyl, naphthyl, anthracyl, furanyl, thiophenyl, quinolyl and pyrrolyl; or a said aryl or hetoroaryl group substituted with 1 to 4 groups chosen from a halogen atom selected from fluorine, chlorine and bromine, linear or branched C₁-C₅ alkyl groups, hydroxy groups, C₁-C₅ alkoxy groups, nitro groups, cyano groups, amino groups, C₁-C₅ monoalkyl amino groups; C₁-C₅ dialkyl amino groups; piperidino groups, piperazino groups or morpholino groups;
- two adjacent of R¹⁰ to R¹³ represent the position or fusion with the oxazine ring in general formula (I), the others having the same meaning as R⁹ and R¹⁴-R¹⁹;
- R⁹ and R¹⁴-R¹⁹ represent a hydrogen atom; a linear or branched C₁-C₅ alkyl group; a C₁-C₅ alkyl group substituted with 1 to 5 atoms of halogen chosen from fluorine chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; a carboxyl group; a C₁-C₅ carboxyalkyl group; a C₁-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluenesulphonic and p-chlorotoluenesulphonic groups; or an aryl group chosen from phenyl, diphenyl and naphthyl groups.

2. Compounds as claimed in claim 1, characterised in that in formula (I):
- R represents a methyl group;
- R¹-R⁴, which are identical or different, each independently represent a hydrogen atom, a fluorine, chlorine or bromine atom, or a methyl, isopropyl, trifluoromethyl, hydroxymethyl, benzyl, hydroxy, methoxy, amino, piperidino, morpholino, carboxyl, carboxymethyl, N,N-dimethylcarboxyamide, cyano, nitro or phenyl group;
- R⁵ and R⁶, both represent a methyl group;
- R⁷ represents a hydrogen atom
- A is one of the groups of formulas (II) to (VI) in which:
- R⁸ represents a hydrogen atom, or a methyl, isopropyl, phenyl, p-N,N-dimethyl aminophenyl, p-cyano phenyl, p-nitro phenyl, p-methoxy phenyl, naphthyl, 2-thiophenyl, 2-furanyl or 4-pyridyl group;
- two adjacent of R¹⁰-R¹³ represent the position of fusion with the oxazine ring in general formula (I), the others then each independently representing a hydrogen atom, a fluorine, chlorine or bromine atom, or a methyl, isopropyl, trifluoromethyl, hydroxymethyl, benzyl, hydroxy, carboxyl, carboxymethyl, N,N-dimethylcarboxyamide, cyano, nitro or phenyl group;
- R⁹ and R¹⁴-R¹⁹ each independently represent a hydrogen atom, a fluorine, chlorine or bromine atom, or a methyl, isopropyl, trifluoromethyl, hydroxymethyl, benzyl, hydroxy, methoxy, carboxyl, carboxymethyl, N,N-dimethylcarboxyamide, cyano, nitro or phenyl group.

3. Photochromatic and thermochromatic compounds according to claim 1, characterised by being:
- 1,3,3-trimethyl-8'-oxophenyl-spiro-indoline-2,3'-[3H]naphtho-(2,1-b)-1,4-oxazine; and
- 1,1,1-trimethyl-spiro-indoline-2,3'-[3H]benzo[a]anthracene-7',12'-dione(3,4-b)-1,4-oxazine.

4. A process for preparing the compounds (I) claimed in claims 1 to 3, characterised by reacting a nitroso derivative of formula (II), (III), (IV), (V) or (VI), in which two adjacent of R¹⁰-R¹³ represent one a -N=O group an the other a -OR²⁰ group where R²⁰ is a hydrogen atom, a metal cation chosen from sodium, potassium, lithium and copper cations; an ammonium or C₁-C₅ dialkylammonium cation; a C₁-C₅ alkylcarbonyl group; a C₁-C₅ alkylsulphonyl group or arylsulphonyl group;
with a compound of formula (VII): 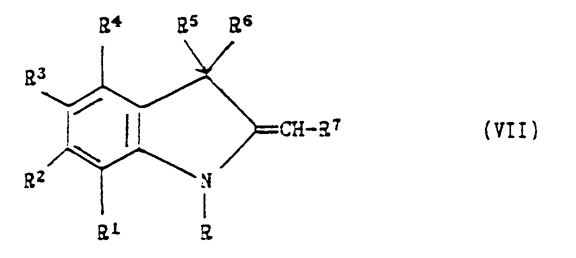 where the substituents R and R¹-R⁷ have the aforesaid meaning.

5. A process as claimed in claim 4, characterised in that the the compound (VII) is added to a solution or suspension of the nitroso derivative of one of the compounds (II), (III), (IV), (V) and (VI), in an inert organic solvent, possibly in the presence of a tertiary amine, operating at a temperature of between 0 and 150°C and preferably between 0 and 80°C, for a time of between about 1 minute and 24 hours.

6. Articles of polymer materials used as solar filters, optical articles, optical memories, prints, photographs, fabrics, decorative articles and toys, characterised by containing at least one photochromatic compound claimed in claims 1 to 3.

## Claims (Claims for the following Contracting State(s): ES)

1. A process for preparing Photochromatic and photosensitizing compounds of the spiro-indoline-oxazine class definable by the general formula (I): 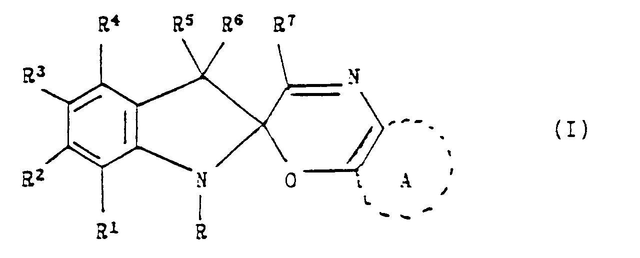 where:
- R represents a methyl group;
- R¹ to R⁴, which can be identical or different, each independently represent a hydrogen atom; a linear or branched C₁-C₅ alkyl group; a said C₁-C₅ alkyl group substituted with 1 to 5 atoms of halogen chosen from fluorine, chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; an amino group; a C₁-C₅ monoxyalkyl amino group; a C₁-C₅ dialkyl amino group; a C₃-C₁₀ cycloalkyl amino group; a piperidino, piperazino or morpholino group; a carboxyl group; a C₁-C₅ carboxyalkyl group; a C₂-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluenesulphonic and p-chlorotoluenesulphonic groups; or an aryl group chosen from phenyl, diphenyl and naphthyl groups;
- R⁵ and R⁶, both represent a methyl group;
- R⁷ represents a hydrogen atom;
- A represents a monocyclic or polycyclic arene group containing at least one carbonyl function (C=O) on the nucleus or in the side chain, and chosen from those representable by the following formulas (II), (III), (IV), (V) or (VI): 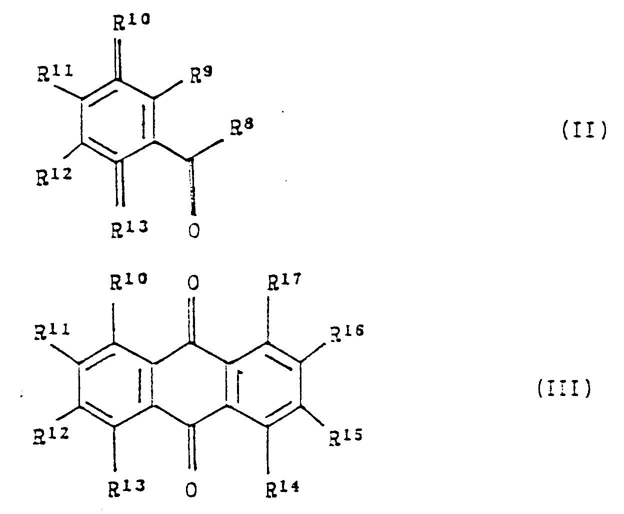 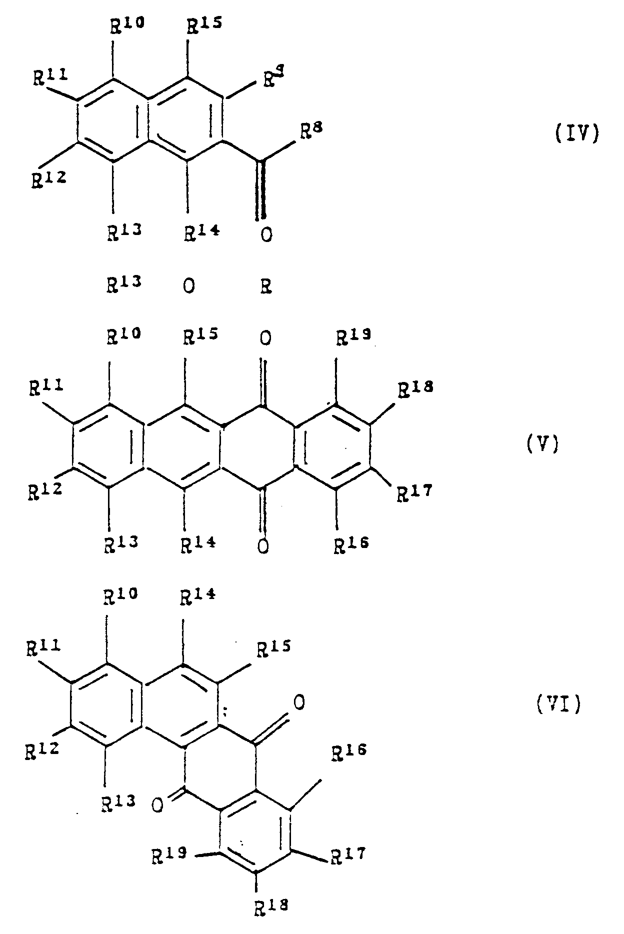 where:
- R⁸ represents a hydrogen atom; a halogen atom chosen from chlorine and bromine; a linear or branched C₁-C₅ alkyl group; a C₂-C₅ alkenyl group; a monocyclic or polycyclic aryl group; or a heteroaryl group chosen from phenyl, naphthyl, anthracyl, furanyl, thiophenyl, quinolyl and pyrrolyl; or a said aryl or heteroaryl group substituted with 1 to 4 groups chosen from a halogen atom selected from fluorine, chlorine and bromine, linear or branched C₁-C₅ alkyl groups, hydroxy groups, C₁-C₅ alkoxy groups, nitro groups, cyano groups, amino groups, C₁-C₅ monoalkyl amino groups; C₁-C₅ dialkyl amino groups; piperidino groups, piperazino groups or morpholino groups;
- two adjacent of R¹⁰ to R¹³ represent the position of fusion with the oxazine ring in general formula (I), the others having the same meaning as R⁹ and R¹⁴-R¹⁹;
- R⁹ and R¹⁴-R¹⁹ represent a hydrogen atom; a linear or branched C₁-C₅ alkyl group; a C₁-C₅ alkyl group substituted with 1 to 5 atoms of halogen chosen from fluorine, chlorine, bromine and iodine, hydroxy groups, C₁-C₅ alkoxy groups, C₁-C₅ carboxyalkyl groups, cyano groups; a C₂-C₅ alkenyl group; a benzyl group; a halogen atom chosen from fluorine, chlorine, bromine and iodine; a hydroxy group; a C₁-C₅ alkoxy group; a carboxyl group; a C₁-C₅ carboxyalkyl group; a C₁-C₅ carboxyalkenyl group; a carboxyamide group; a C₁-C₅ N-alkyl substituted carboxyamide group; a C₁-C₅ N,N-dialkyl substituted carboxyamide group; a cyano group; a nitro group; a sulphonic group; a C₁-C₅ alkylsulphonic group; an arylsulphonic group chosen from benzenesulphonic, p-toluenesulphonic and p-chlorotoluenesulphonic groups; or an aryl group chosen from phenyl, diphenyl and naphthyl groups;
said process comprising the step of:
reacting a nitroso derivative of formula (II), (III), (IV), (V) or (VI), in which two adjacent of R¹⁰-R¹³ represent one a -N=O group an the other a -OR²⁰ group where R²⁰ is a hydrogen atom, a metal cation chosen from sodium, potassium, lithium and copper cations; an ammonium or C₁-C₅ dialkylammonium cation; a C₁-C₅ alkylcarbonyl group; a C₁-C₅ alkylsulphonyl group or arylsulphonyl group;
with a compound of formula (VII): 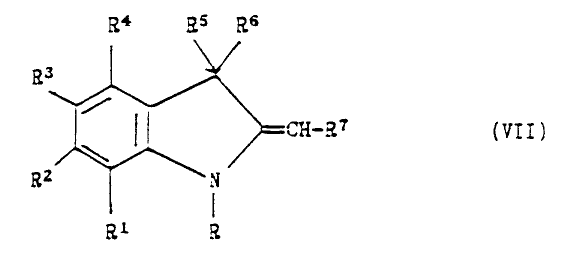 where the substituents R and R¹-R⁷ have the aforesaid meaning.

2. A process as claimed in claim 1, characterised in that in formula (I):
- R represents a methyl, group;
- R¹-R⁴, which are identical or different, each independently represent a hydrogen atom, a fluorine, chlorine or bromine atom, or a methyl, isopropyl, trifluoromethyl, hydroxymethyl, benzyl, hydroxy, methoxy, amino, piperidino, morpholino, carboxyl, carboxymethyl, N,N-dimethylcarboxyamide, cyano, nitro or phenyl group;
- R⁵ and R⁶, both represent a methyl group;
- R⁷ represents a hydrogen atom;
- A is one of the groups of formulas (II) to (VI) in which:
- R⁸ represents a hydrogen atom, or a methyl, isopropyl, phenyl, p-N,N-dimethyl aminophenyl, p-cyano phenyl, p-nitro phenyl, p-methoxy phenyl, naphthyl, 2-thiophenyl, 2-furanyl or 4-pyridyl group;
- two adjacent of R¹⁰-R¹³ represent the position of fusion with the oxazine ring in general formula (I), the others then each independently representing a hydrogen atom, a fluorine, chlorine or bromine atom, or a methyl, isopropyl, trifluoromethyl, hydroxymethyl, benzyl, hydroxy, carboxyl, carboxymethyl, N,N-dimethylcarboxyamide, cyano, nitro or phenyl group;
- R⁹ and R¹⁴-R¹⁹ each independently represent a hydrogen atom, a fluorine, chlorine or bromine atom, or a methyl, isopropyl, trifluoromethyl, hydroxymethyl, benzyl, hydroxy, methoxy, carboxyl, carboxymethyl, N,N-dimethylcarboxyamide, cyano, nitro or phenyl group.

3. A process according to claim 1, characterised in that the photochromatic and thermochromatic compounds are:
- 1,3,3-trimethyl-8'-oxophenyl-spiro-indoline-2,3'-[3H]naphtho-(2,1-b)-1,4-oxazine; and
- 1,1,1-trimethyl-spiro-indoline-2,3'-[3H]benzo[a]anthracene-7',12'-dione(3,4-b)-1,4-oxazine.

4. A process as claimed in claim 1, characterised in that the the compound (VII) is added to a solution or suspension of the nitroso derivative of one of the compounds (II), (III), (IV), (V) and (VI), in an inert organic solvent, possibly in the presence of a tertiary amine, operating at a temperature of between 0 and 150°C and preferably between 0 and 80°C, for a time of between about 1 minute and 24 hours.

5. A process for preparing Articles of polymer materials used as solar filters, optical articles, optical memories, prints, photographs, fabrics, decorative articles and toys, characterised by containing at least one photochromatic compound prepared as in claims 1 to 3.

## Patentansprüche (Patentansprüche für folgende(n) Vertragsstaat(en): AT, BE, CH, DE, DK, FR, GB, GR, LI, LU, NL, SE)

1. Photochromatische und photoempfindliche Verbindungen der Spiroindolinoxazinreihe, definierbar durch die allgemeine Formel (I): 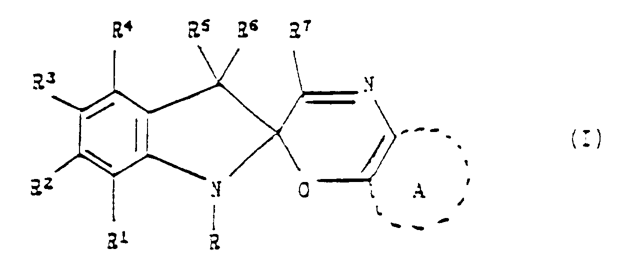
- in der R eine Methylgruppe bedeutet;
- R¹ bis R⁴, die gleich oder verschieden sein können, jeweils unabhängig ein Wasserstoffatom, eine linear oder verzweigte C₁-C₅-Alkylgruppe; eine solche C₁-C₅-Alkylgruppe, die substituiert ist mit 1 bis 5 Halogenatomen, ausgewählt aus Fluor, Chlor, Brom und Iod, Hydroxygruppen, C₁-C₅-Alkoxygruppen, C₁-C₅-Carboxyalkylgruppen oder Cyanogruppen; eine C₂-C₅-Alkenylgruppe; eine Benzylgruppe; ein Halogenatom, ausgewählt aus Fluor, Chlor, Brom und Iod; eine Hydroxygruppe; eine C₁-C₅-Alkoxygruppe; eine Aminogruppe; eine C₁-C₅-Monooxyalkylaminogruppe; eine C₁-C₅-Dialkylaminogruppe; eine C₃-C₁₀-Cycloalkylaminogruppe; eine Piperidino-, Piperazino- oder Morpholinogruppe; eine Carboxylgruppe; eine C₁-C₅-Carboxyalkylgruppe; eine C₂-C₅-Carboxyalkenylgruppe; eine Carboxyamidgruppe; eine C₁-C₅-N-Alkyl substituierte-Carboxydamidgruppe; eine C₁-C₅-N,N-Dialkyl-substituierte-Carboxyamidgruppe; eine Cyanogruppe; eine Nitrogruppe; eine Sulfonsäuregruppe; eine C₁-C₅-Alkylsulfonsäuregruppe; eine Arylsulfonsäuregruppe, ausgewählt aus Benzolsulfonsäure-, p-Toluolsulfonsäure- und p-Chlortoluolsulfonsäuregruppe oder eine Arylgruppe, ausgewählt aus Phenyl-, Diphenyl- und Naphthylgruppen bedeuten;
- R⁵ und R⁶, jeweils eine Methylgruppe bedeuten;
- R⁷ ein Wasserstoffatom bedeutet;
- A eine monocyclische oder polycyclische Arengruppe, enthaltend mindestens eine Carbonylfunktion (C=O) am Kern oder in der Seitenkette und ausgewählt aus den folgenden Formeln (II), (III), (IV), (V) oder (VI) bedeutet 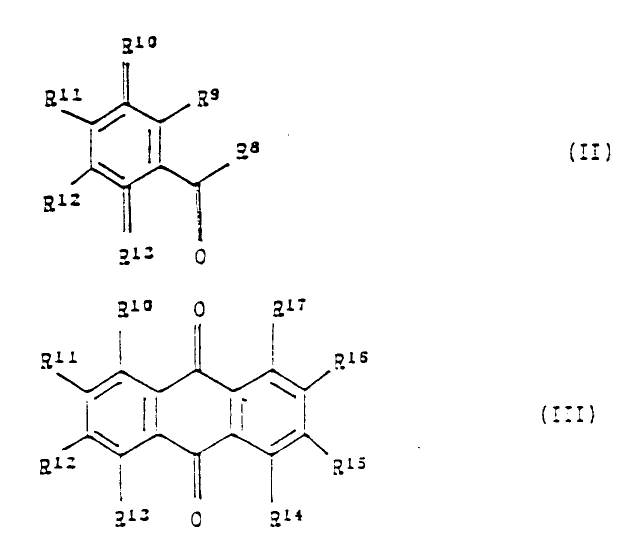 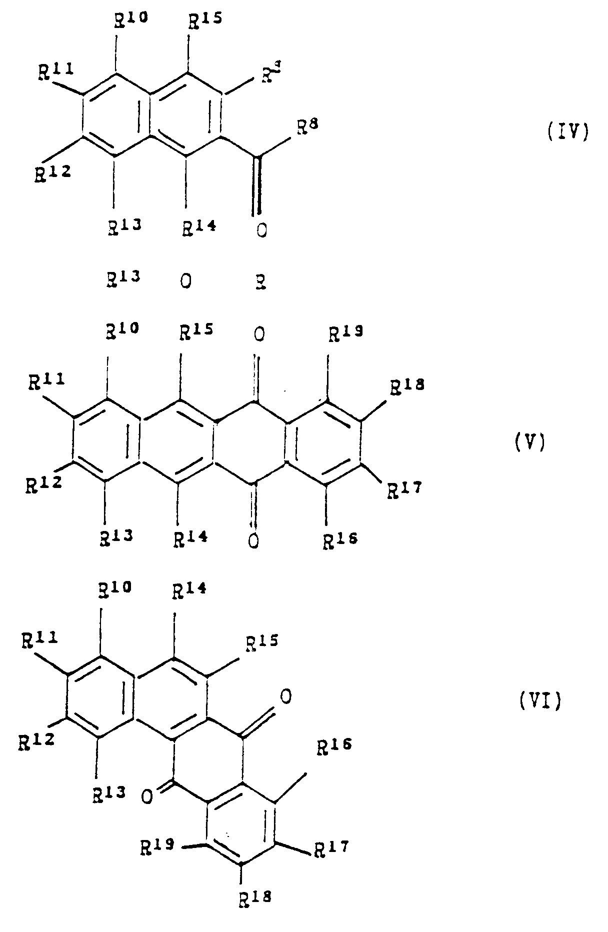 wobei
- R⁸ ein Wasserstoffatom; ein Halogenatom, ausgewählt aus Chlor und Brom; eine lineare oder verzweigte C₁-C₅-Alkylgruppe, eine C₂-C₅-Alkenylgruppe; eine monocyclische oder polyzyclische Arylgruppe oder eine Heteroarylgruppe, ausgewählt aus Phenyl, Naphthyl, Anthracyl, Furanyl, Thiophenyl, Chinolyl und Pyrrolyl oder eine solche Aryl- oder Heteoarylgruppe, die substituiert ist mit 1 bis 4 Gruppen, ausgewählt aus einem Halogenatom, ausgewählt aus Fluor, Chlor und Brom, linearen oder verzweigten C₁-C₅-Alkylgruppen, Hydroxygruppen; C₁-C₅-Alkoxygruppen, Nitrogruppen, Cyanogruppen, Aminogruppen, C₁-C₅-Monoalkylaminogruppen; C₁-C₅-Dialkylaminogruppen; Piperidinogruppen, Piperazinogruppen oder Morpholinogruppen, bedeutet
- zwei benachbarte R¹⁰ bis R¹³, die Kondensationsstellen mit den in der allgemeinen Formel (I) angegebenen Oxazinring darstellen und die anderen die gleiche Bedeutung haben wie R⁹ und R¹⁴ bis R¹⁹ ;
- R⁹ und R¹⁴ bis R¹⁹ ein Wasserstoffatom; eine lineare oder verzweigte C₁-C₅-Alkylgruppe, eine C₁-C₅-Alkylgruppe, substituiert mit 1 bis 5 Halogenatomen, ausgewählt aus Fluor, Chlor, Brom oder Iod, Hydroxygruppen, C₁-C₅-Carboxylalkylgruppen oder Cyanogruppen; eine C₂-C₅-Alkenylgruppe; eine Benzylgruppe; ein Halogenatom, ausgewählt aus Fluor, Chlor, Brom und Iod; eine Hydroxygruppe; eine C₁-C₅-Alkoxygruppe; eine Carboxylgruppe; eine C₁-C₅-Carboxyalkylgruppe; eine C₁-C₅-Carboxyalkenylgruppe; eine Carboxyamidgruppe; eine C₁-C₅-N-Alkyl substituierte-Carboxyamidgruppe; eine C₁-C₅-N,N-Dialkyl-substituierte-Carboxyamidgruppe; eine Cyanogruppe; eine Nitrogruppe; eine Sulfonsäuregruppe; eine C₁-C₅-Alkylsulfonsäuregruppe; eine Arylsulfonsäuregruppe, ausgewählt aus Benzolsulfonsäure-, p-Toluolsulfonsäure- und p-Chlortoluolsulfonsäuregruppen oder eine Arylgruppe, ausgewählt aus Phenyl-, Diphenyl- und Naphthylgruppen bedeuten.

2. Verbindungen nach Anspruch 1,
dadurch gekennzeichnet, daß in der Formel (I)
- R eine Methylgruppe bedeutet;
- R¹ bis R⁴, die gleich oder verschieden sein können, jeweils unabhängig ein Wasserstoffmatom, ein Fluor-, Chlor- oder Bromatom oder eine Methyl-, Isopropyl-, Trifluormethyl-, Hydroxymethyl-, Benzyl, Hydroxy-, Methoxy-, Amino-, Piperidino-, Morpholino-, Carboxyl-, Carboxylmethyl-, N,N-Dimethylcarboxyamid-, Cyano-, Nitro- oder Phenylgruppe bedeuten,
- R⁵ und R⁶ jeweils eine Methylgruppe bedeuten,
- R⁷ ein Wasserstoffatom bedeutet;
- A eine der Gruppen der Formeln (II) bis (VI) ist, wobei
- R⁸ ein Wasserstoffatom oder eine Methyl-, Isopropyl-, Phenyl-, p-N,N-Dimethylaminophenyl, p-Cyanophenyl-, p-Nitrophenyol-, p-Methoxyphenyl-, Naphthyl-, 2-Thiophenyl-, 2-Furanyl- oder 4-Pyridylgruppe bedeutet;
- zwei benachbarte Gruppen R¹⁰ bis R¹³ die Kondensationsstelle mit dem Oxazinring in der allgemeinen Formel (I) darstellen und die anderen dann jeweils unabhängig ein Wasserstoffatom, ein Fluor-, Chlor oder Bromatom oder eine Methyl-, Isopropyl-, Trifluormethyl-, Hydroxymethyl-, Benzyl-, Hydroxy-, Carboxyl-, Carboxymethyl-, N,N-Dimethylcarboxyamid-, Cyano-, Nitro- oder Phenylgruppe bedeuten;
- R⁹ und R¹⁴ bis R¹⁹ jeweils unabhängig ein Wasserstoffatom, ein Fluor-, Chlor- oder Bromatom oder eine Methyl-, Isopropyl-, Trifluormethyl-, Hydroxymethyl-, Benzyl-, Hydroxy-, Methoxy-, Carboxyl-, Carboxymethyl-, N,N-Dimethylcarboxyamid-, Chyano-, Nitro- oder Phenylgruppe bedeuten.

3. Photochromatische und thermochromatische Verbindungen nach Anspruch 1,
dadurch gekennzeichnet, daß sie sind
- 1,3,3-Trimethyl-8'-oxophenyl-spiro-indolin-2,3'- 3H naphtho(2,1-b)-1,4-oxazin und
- 1,1,1-Trimethyl-spiro-indolin-2,3'- 3H benzo a anthracen-7',12'-dione(3,4-b)-1,4-oxazin.

4. Verfahren zur Herstellung der Verbindungen (I) nach den Ansprüchen 1 bis 3, gekennzeichnet durch die Umsetzung eines Nitrosoderivats der Formel (II), (III), (IV), (V) oder (VI), bei dem zwei benachbarte Gruppen R¹⁰ bis R¹³ eine -N=0-Gruppe bedeuten und die andere eine -OR²⁰-Gruppe, wobei R²⁰ ein Wasserstoffatom, ein Metallkation, ausgewählt aus Natrium, Kalium, Lithium und Kupferkationen, ein Amonium oder C₁-C₅ Dialkylamoniumkation, eine C₁-C₅ Alkylcarbonylgruppe, eine C₁-C₅ Alkylsulfonylgruppe oder Arylsulfonylgruppe bedeutend mit einer Verbindung der Formel (VII) 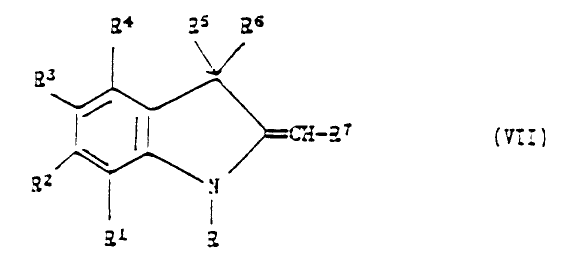 wobei die substituenden R und R¹ und R⁷ die oben angegebene Bedeutung haben.

5. Verfahren nach Anspruch 4,
dadurch gekennzeichnet, daß die Verbindung (VII) zu einer Lösung oder Suspension des Nitrosoderivats einer der Verbindungen (II), (III), (IV), (V) und (VI) in einem inerten organischen Lösungsmittel, gegebenenfalls in Gegenwart eines tertiären Amins, zugegeben wird, bei einer Temperatur zwischen 0 und 150°C, vorzugsweise zwischen 0 und 80°C, während einer Zeit zwischen etwa 1 Minute und 24 Stunden gearbeitet wird.

6. Gegenstand aus Polymermaterialien, der angewandt wird für Sonnenfilter, optische Gegenstände, optische Memoryeinheiten, Drucke, Photographien, Stoffe, Dekorationsartikel und Spielzeuge,
dadurch gekennzeichnet, daß er mindestens eine photochromatische Verbindung nach den Ansprüchen 1 bis 3 enthält.

## Patentansprüche (Patentansprüche für folgende(n) Vertragsstaat(en): ES)

1. Verfahren zur Herstellung von photochromatischen und photoempfindlichen Verbindungen der Spiroindolinoxazinreihe, definierbar durch die allgemeine Formel (I): 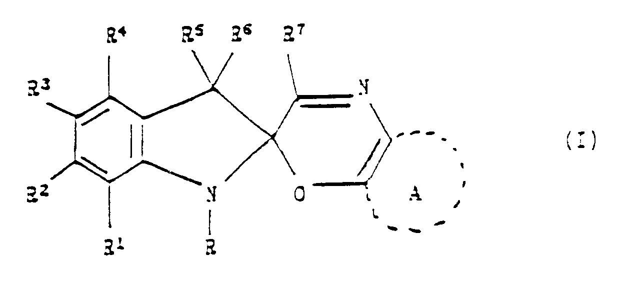
- in der R eine Methylgruppe bedeutet;
- R¹ bis R⁴, die gleich oder verschieden sein können, jeweils unabhängig ein Wasserstoffatom, eine linear oder verzweigte C₁-C₅-Akylgruppe; eine solche C₁-C₅-Alkylgruppe, die substituiert ist mit 1 bis 5 Halogenatomen, ausgewählt aus Fluor, Chlor, Brom und Iod, Hydroxygruppen, C₁-C₅-Alkoxygruppen, C₁-C₅-Carboxyalkylgruppen oder Cyanogruppen; eine C₂-C₅ Alkenylgruppe; eine Benzylgruppe; ein Halogenatom, ausgewählt aus Fluor, Chlor, Brom und Iod; eine Hydroxygruppe, eine C₁-C₅-Alkoxygruppe; eine Aminogruppe; eine C₁-C₅-Monooxyalkylaminogruppe; eine C₁-C₅-Dialkylaminogruppe, eine C₃-C₁₀-Cycloalkylaminogruppe; eine Piperidino-, Piperazino- oder Morpholinogruppe; eine Carboxylgruppe; eine C₁-C₅-Carboxlalkylgruppe; eine C₂-C₅-Carboxyalkenylgruppe; eine Carboxyamidgruppe; eine C₁-C₅-N-Alkyl-substituierte-Carboxydamidgruppe; eine C₁-C₅-N,N-Dialkyl-substituierte-Carboxyamidgruppe; eine Cyanogruppe; eine Nitrogruppe; eine Sulfonsäuregruppe; eine C₁-C₅-Alkylsulfonsäuregruppe; eine Arylsulfonsäuregruppe, ausgewählt aus Benzolsulfonsäure-, p-Toluolsulfonsäure- und p-Chlortoluolsulfonsäuregruppe oder eine Arylgruppe, ausgewählt aus Phenyl, Diphenyl und Naphthylgruppen bedeuten; - R⁵ und R⁶, jeweils eine Methylgruppe bedeuten; - R⁷ ein Wasserstoffatom bedeutet;
- A eine monocyclische oder polyzyclische Arengruppe, enthaltend mindestens eine Carbonylfunktion (C=0) am Kern oder in der Seitenkette und ausgewählt aus den folgenden Formeln (II), (III), (IV), (V) oder (VI) bedeutet 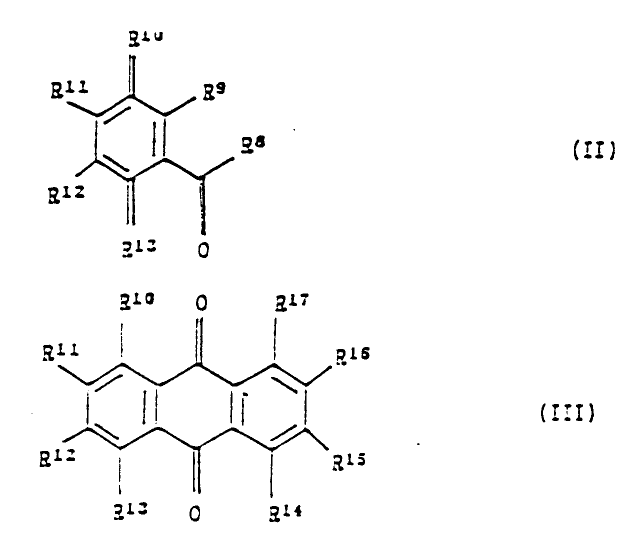 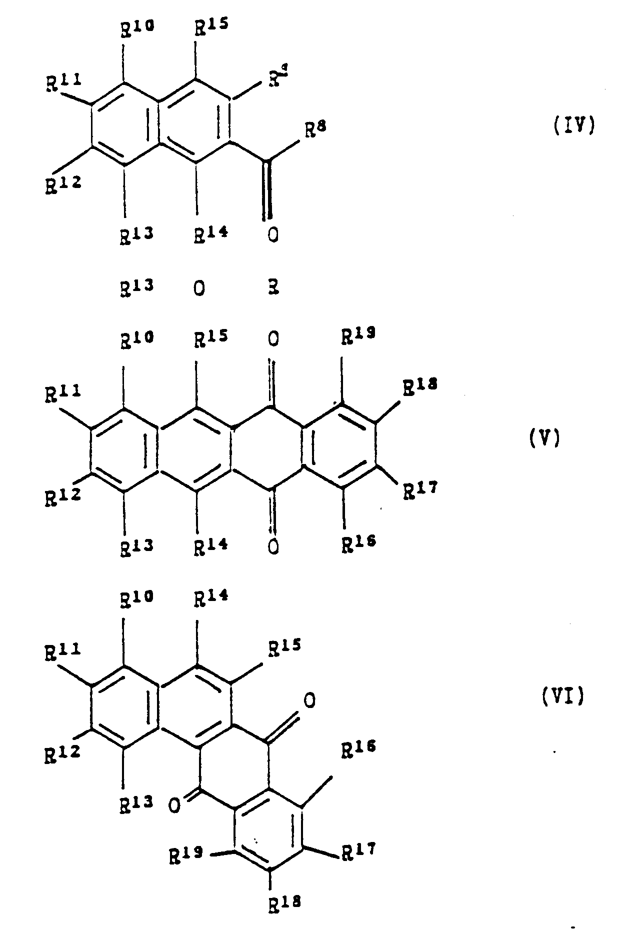 wobei
- R⁸ ein Wasserstoffatom; ein Halogenatom, ausgewählt aus Chlor und Brom; eine lineare oder verzweigte C₁-C₅-Alkylgruppe, eine C₂-C₅-Alkenylgruppe; eine monocyclische oder polycyclische Arylgruppe oder eine Heteroarylgruppe, ausgewählt aus Phenyl, Naphthyl, Anthracyl, Furanyl Thiophenyl, Chinolyl und Pyrrolyl oder eine solche Aryl oder Heteroarylgruppe, die substituiert ist mit 1 bis 4 Gruppen, ausgewählt aus einem Halogenatom, ausgewählt aus Fluor, Chlor und Brom, linearen oder verzweigten C₁-C₅-Alkylgruppen; Hydroxygruppen; C₁-C₅-Alkoxygruppen, Nitrogruppen, Cyanogruppen, Aminogruppen, C₁-C₅-Monoalkylaminogruppen; C₁-C₅-Dialkylaminogruppen; Piperidinogruppen, Piperazinogruppen oder Morpholinogruppen, bedeutet
- zwei benachbarte R¹⁰ bis R¹³, die Kondensationsstellen mit den in der allgemeinen Formel (I) angegebenen Oxazinring darstellen und die anderen die gleiche Bedeutung haben wie R⁹ und R¹⁴ bis R¹⁹;
- R⁹ und R¹⁴ bis R¹⁹ ein Wasserstoffatom; eine lineare oder verzweigte C₁-C₅-Alkylgruppe; eine C₁-C₅-Alkylgruppe, substituiert mit 1 bis 5 Halogenatomen, ausgewählt aus Fluor, Chlor, Brom und Iod, Hydroxygruppen, C₁-C₅-Carboxylalkylgruppen oder Cyanogruppen; eine C₂-C₅-Alkenylgruppe; eine Benzylgruppe; ein Halogenatom, ausgewählt aus Fluor, Chlor, Brom und Iod; eine Hydroxygruppe; eine C₁-C₅-Alkoxygruppe; eine Carboxylgruppe; eine C₁-C₅-Carboxylalkylgruppe, eine C₁-C₅-Carboxyalkenylgruppe; eine Carboxyamidgruppe; eine C₁-C₅-N-Alkyl-substituierte-Carboxyamidgruppe; eine C₁-C₅-N,N-Dialkyl substituierte-Carboxyamidgruppe; eine Cyanogruppe; eine Nitrogruppe, eine Sulfonsäuregruppe; eine C₁-C₅-Alkylsulfonsäuregruppe, eine Arylsulfonsäuregruppe, ausgewählt aus Benzolsulfonsäure-, p-Toluolsulfonsäure- und p-Chlortoluolsulfonsäuregruppen oder eine Arylgruppe, ausgewählt aus Phenyl-, Diphenyl- und Naphthylgruppen bedeuten, wobei das Verfahren umfaßt
- die Stufe der Umsetzung eines Nitrosoderivats der Formel (II), (III), (IV), (V) oder (VI), bei dem zwei benachbarte Gruppen R¹⁰ bis R¹³ eine -N=0-Gruppe bedeuten und die andere eine -OR²⁰-Gruppe, wobei R²⁰ ein Wasserstoffatom, ein Metallkation, ausgewählt aus Natrium, Kalium, Lithium und Kupferkationen, ein Amonium oder C₁-C₅-Dialkylamoniumkation, eine C₁-C₅-Alkylcarbononylgruppe, eine C₁-C₅-Alkylsulfonylgruppe oder Arylsulfonylgruppe bedeutend mit einer Verbindung der Formel (VII) 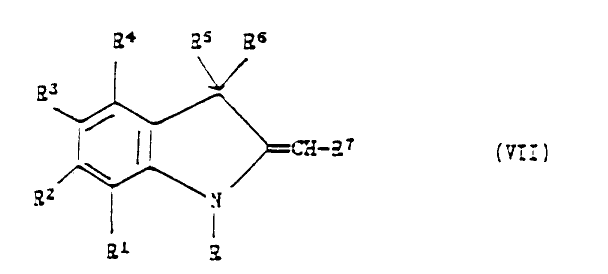 wobei die substituenden R und R¹ und R⁷ die oben angegebene Bedeutung haben.

2. Verfahren nach Anspruch 1,
dadurch gekennzeichnet, daß bei der Formel (I)
- R eine Methylgruppe bedeutet;
- R¹ bis R⁴, die gleich oder verschieden sein können, jeweils unabhängig ein Wasserstoffatom, ein Fluor-, Chlor- oder Bromatom oder eine Methyl-, Isopropyl-, Trifluormethyl-, Hydroxymethyl-, Benzyl-, Hydroxy-, Methoxy-, Amino-, Piperidino-, Morpholino-, Carboxyl-, Carboxylmethyl-, N,N-Dimethylcarboxyamid-, Cyano-, Nitro- oder Phenylgruppe bedeuten,
- R⁵ und R⁶ jeweils eine Methylgruppe bedeuten,
- R⁷ ein Wasserstoffatom bedeutet;
- A eine der Gruppen der Formeln (II bis VI) ist, wobei
- R⁸ ein Wasserstoffatom oder eine Methyl-, Isopropyl-, Phenyl-, p-N,N-Dimethylaminophenyl-, p-Cyanophenyl-, p-Nitrophenyol-, p-Methoxyphenyl-, Naphthyl-, 2-Thiophenyl-, 2-Fruanyl- oder 4-Pyridylgruppe bedeutet;
- zwei benachbarte Gruppen R¹⁰ bis R¹³ die Kondensationsstelle mit dem Oxazinring in der allgemeinen Formel (I) darstellen und die anderen dann jeweils unabhängig ein Wasserstoffatom, ein Fluor-, Chlor- oder Bromatom oder eine Methyl-, Isopropyl-, Trifluormethyl-, Hydroxymethyl-, Benzyl-, Hydroxy-, Carboxyl-, Carboxymethyl-, N,N-Dimethylcarboxyamid-, Cyano-, Nitro- oder Phenylgruppe bedeuten;
- R⁹ und R¹⁴ bis R¹⁹ jeweils unabhängig ein Wasserstoffatom, ein Fluor-, Chlor- oder Bromatom oder eine Methyl-, Isopropyl, Trifluormethyl-, Hydroxymethyl-, Benzyl-, Hydroxy-, Methoxy-, Carboxyl-, Carboxymethyl-, N,N-Dimethylcarboxyamid-, Cyano-, Nitro- oder Phenylgruppe bedeuten.

3. Verfahren nach Anspruch 1,
dadurch gekennzeichnet, daß die photochromatischen und thermochromatischen Verbindungen sind
- 1,3,3-Trimethyl-8'-oxophenyl-spiro-indolin-2,3'- 3H naphtho(2,1-b)-1,4-oxazin und
- 1,1,1-Trimethyl-spiro-indolin-2,3'- 3H benzo a anthracen-7',12'-dione(3,4-b)-1,4-oxazin.

4. Verfahren nach Anspruch 1,
dadurch gekennzeichnet, daß die Verbindung (VII) zu einer Lösung oder Suspension des Nitrosoderivats einer der Verbindungen (II), (III), (IV), (V) und (VI) in einem inerten organischen Lösungsmittel, gegebenenfalls in Gegenwart eines tertiären Amins zugegeben wird, bei einer Temperatur zwischen 0 und 150°C, vorzugsweise zwischen 0 und 80°C, während einer Zeit zwischen etwa 1 Minute und 24 Stunden gearbeitet wird.

5. Verfahren zur Herstellung von Gegenständen aus polymeren Materialien, die verwendet werden als Sonnenfilter, optische Gegenstände, optische Memoryeinheiten, Drucke, Photographien, Stoffe, Dekorationsartikel und Spielzeuge,
dadurch gekennzeichnet, daß sie mindestens eine photochromatische Verbindung, hergestellt wie in den Ansprüchen 1 bis 3 angegeben, enthalten.

## Revendications (Revendications pour l'(les) Etat(s) contractant(s) suivant(s): AT, BE, CH, DE, DK, FR, GB, GR, LI, LU, NL, SE)

1. Composés photochromes et photosensibles de la classe des spiro-indoline-oxazines pouvant être définis par la formule générale (I) : 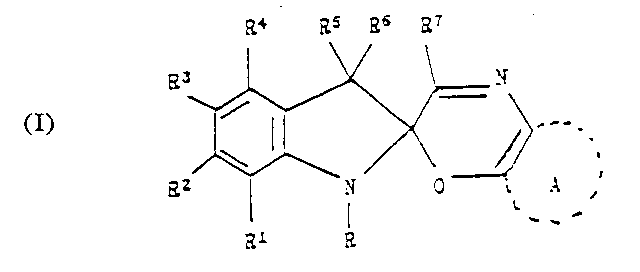 dans laquelle
R représente un groupe méthyle,
R¹ à R⁴, identiques ou différents, représentent chacun indépendamment un atome d'hydrogène, un groupe alkyle en C₁₋₅ à chaîne linéaire ou ramifiée, un tel groupe alkyle en C₁₋₅ substitué par 1 à 5 atomes d'halogène choisis parmi le fluor, le chlore, le brome et l'iode, ou par 1 à 5 groupes hydroxy, alcoxy en C₁₋₅, carboxyalkyle en C₁₋₅ ou cyano, un groupe alcényle en C₂₋₅, un groupe benzyle, un atome d'halogène choisi parmi le fluor, le chlore, le brome et l'iode, un groupe hydroxy, un groupe alcoxy en C₁₋₅, un groupe amino, un groupe (monoxyalkyle en C₁₋₅)-amino, un groupe di(alkyle en C₁₋₅)amino, un groupe (cycloalkyle en C₃₋₁₀)amino, un groupe pipéridino, pipérazino ou morpholino, un groupe carboxyle, un groupe carboxy(alkyle en C₁₋₅), un groupe carboxy(alcényle en C₂₋₅), un groupe carboxyamide, un groupe N-(alkyle en C₁₋₅)-carboxyamide, un groupe N,N-di(alkyle en C₁₋₅)carboxyamide, un groupe cyano, un groupe nitro, un groupe sulfonique, un groupe (alkyle en C₁₋₅)sulfonique, un groupe arylsulfonique choisi parmi les groupes benzènesulfonique, p-toluènesulfonique et p-chlorotoluènesulfonique, ou un groupe aryle choisi parmi les résidus phényle, diphényle et naphtyle,
R⁵ et R⁶ représentent tous les deux un groupe méthyle,
A représente un groupe arène monocyclique ou polycyclique contenant au moins une fonction carbonyle (C=O) sur le noyau ou dans la chaîne latérale, et qui est choisi parmi ceux représentés par les formules (II), (III), (IV), (V) ou (VI) suivantes : 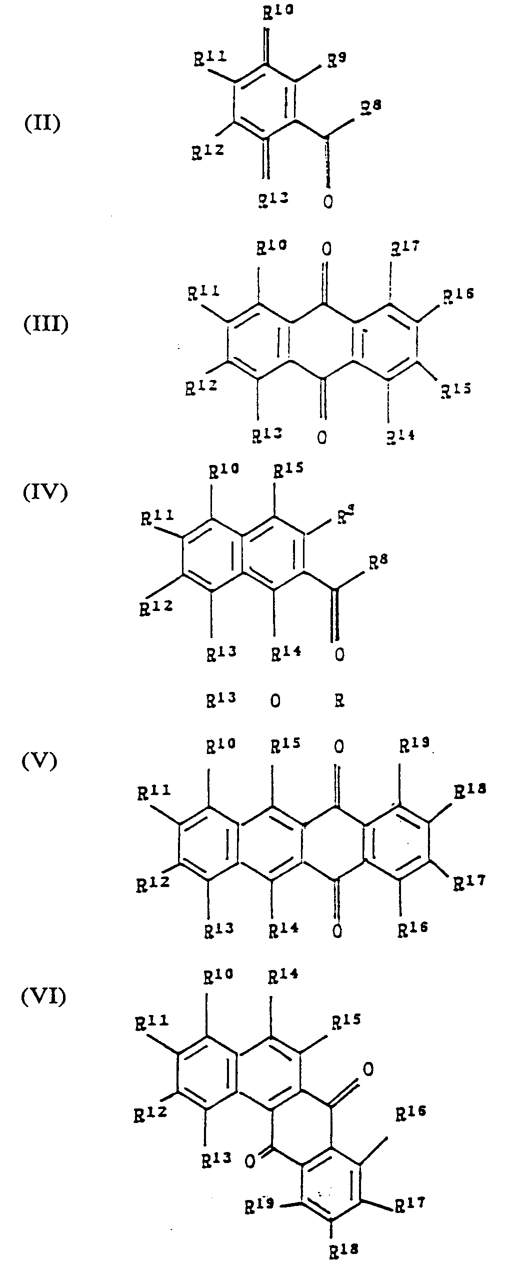 dans lesquelles
R⁸ représente un atome d'hydrogène, un atome d'halogène choisi parmi le chlore et le brome, un groupe alkyle en C₁₋₅ à chaîne linéaire ou ramifiée, un groupe alcényle en C₂₋₅, un groupe aryle monocyclique ou polycyclique, ou un groupe hétéroaryle choisi parmi les résidus phényle, naphtyle, anthracyle, furanyle, thiophényle, quinolyle et pyrrolyle, ou un tel groupe aryle ou hétéroaryle substitué par 1 à 4 groupes choisis parmi les atomes d'halogène tel que le fluor, le chlore et le brome, les groupes alkyle en C₁₋₅ à chaîne linéaire ou ramifiée, hydroxy, alcoxy en C₁₋₅, nitro, cyano, amino, mono(alkyle en C₁₋₅)amino, di(alkyle en C₁₋₅)amino, pipéridino, pipérazino ou morpholino,
deux résidus adjacents parmi les résidus R¹⁰ à R¹³ représentent les positions de fusion avec le noyau oxazine dans la formule générale (I), les autres ayant la même signification que R⁹ et R¹⁴ à R¹⁹,
R⁹ et R¹⁴ à R¹⁹ représentent un atome d'hydrogène, un groupe alkyle en C₁₋₅ à chaîne linéaire ou ramifiée, un groupe alkyle en C₁₋₅ substitué par 1 à 5 atomes d'halogène choisis parmi le fluor, le chlore, le brome et l'iode, par 1 à 5 groupes hydroxy, alcoxy en C₁₋₅, carboxy(alkyle en C₁₋₅) ou cyano, un groupe alcényle en C₂₋₅, un groupe benzyle, un atome d'halogène choisi parmi le fluor, le chlore, le brome et l'iode, un groupe hydroxy, un groupe alcoxy en C₁₋₅, un groupe carboxyle, un groupe carboxy(alkyle en C₁₋₅), un groupe carboxy(alcényle en C₂₋₅), un groupe carboxyamide, un groupe N-(alkyle en C₁₋₅)-carboxyamide, un groupe N,N-di(alkyle en C₁₋₅)carboxyamide, un groupe cyano, un groupe nitro, un groupe sulfonique, un groupe (alkyle en C₁₋₅)sulfonique, un groupe arylsulfonique choisi parmi les groupes benzènesulfonique, p-toluènesulfonique et p-chlorotoluènesulfonique, ou un groupe aryle choisi parmi les résidus phényle, diphényle et naphtyle.

2. Composés conformes à la revendication 1, caractérisés en ce que dans la formule (I)
R représente un groupe méthyle,
R¹ à R⁴, identiques ou différents, représentent chacun indépendamment un atome d'hydrogène, un atome de fluor, de chlore ou de brome, ou un groupe méthyle, isopropyle, trifluorométhyle, hydroxyméthyle, benzyle, hydroxy, méthoxy, amino, pipéridino, morpholino, carboxyle, carboxyméthyle, N,N-diméthylcarboxyamide, cyano, nitro ou phényle,
R⁵ et R⁶ représentent tous les deux un groupe méthyle,
R⁷ représente un atome d'hydrogène,
A correspond à un des groupes de formule (II) à (VI) dans lesquelles R⁸ représente un atome d'hydrogène, ou un groupe méthyle, isopropyle, phényle, p-N,N-diméthylaminophényle, p-cyanophényle, p-nitrophényle, p-méthoxyphényle, naphtyle, 2-thiophényle, 2-furanyle ou 4-pyridyle,
deux résidus adjacents parmi les résidus R¹⁰ à R¹³ représentent les positions de fusion avec le noyau oxazine dans la formule générale (I), les autres représentant indépendamment un atome d'hydrogène, un atome de fluor, de chlore ou de brome, ou un groupe méthyle, isopropyle, trifluorométhyle, hydroxyméthyle, benzyle, hydroxy, carboxy, carboxyméthyle, N,N-diméthylcarboxyamide, cyano, nitro ou phényle,
R⁹ et R¹⁴⁻¹⁹ représentent chacun indépendamment un atome d'hydrogène, un atome de fluor, de chlore ou de brome, ou un groupe méthyle, isopropyle, trifluorométhyle, hydroxyméthyle, benzyle, hydroxy, méthoxy, carboxyle, carboxyméthyle, N,N-diméthylcarboxyamide, cyano, nitro ou phényle.

3. Composés photochromes et thermochromes conformes à la revendication 1, caractérisés en ce qu'il s'agit
de la 1,3,3-triméthyl-8'-oxophényl-spiro-indoline-2,3'-[3H]naphto(2,1-b)-1,4-oxazine, et
de la 1,1,1-triméthyl-spiro-indoline-2,3'-[3H]benzo[a]anthracène-7',12'-dione(3,4-b)-1,4-oxazine.

4. Procédé de préparation des composés (I) conformes aux revendications 1 à 3, caractérisé en ce que l'on fait réagir un dérivé nitroso des composés de formule (II), (III), (IV), (V) ou (VI) dans lesquelles un de deux résidus adjacents des groupes R¹⁰ à R¹³ représente un groupe -N=O et l'autre un groupe -OR²⁰, où R²⁰ représente un atome d'hydrogène, un cation métallique choisi parmi le sodium, le potassium le lithium et le cuivre, un cation ammonium ou di(alkyle en C₁₋₅)ammonium, un groupe (alkyle en C₁₋₅)carbonyle, un groupe (alkyle en C₁₋₅)sulfonyle ou un groupe arylsulfonyle,
avec un composé de formule (VII) 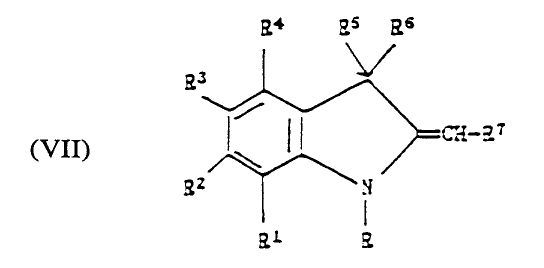 dans laquelle les substituants R et R¹ à R⁷ ont la signification indiquée ci-avant.

5. Procédé conforme à la revendication 4, caractérisé en ce que le composé (VII) est ajouté à une solution ou suspension du dérivé nitroso d'un des composés de formule (II), (III), (IV), (V) ou (VI), dans un solvant organique inerte, éventuellement en présence d'une amine tertiaire, en opérant à une température comprise entre 0 et 150 °C et de préférence entre 0 et 80 °C, pendant une période comprise entre environ 1 minute et 24 heures.

6. Articles en un matériau polymère utilisés comme filtres solaires, articles optiques, mémoires optiques, imprimés, photographies, tissus, articles décoratifs et jouets, caractérisés en ce qu'ils contiennent au moins un composé photochrome conforme aux revendications 1 à 3.

## Revendications (Revendications pour l'(les) Etat(s) contractant(s) suivant(s): ES)

1. Procédé permettant de préparer des composés photochromes et photosensibles de la classe des spiro-indoline-oxazines pouvant être définis par la formule générale (I) : 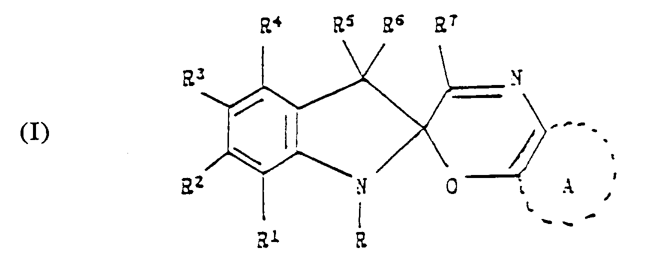 dans laquelle
R représente un groupe méthyle,
R¹ à R⁴, identiques ou différents, représentent chacun indépendamment un atome d'hydrogène, un groupe alkyle en C₁₋₅ à chaîne linéaire ou ramifiée, un tel groupe alkyle en C₁₋₅ substitué par 1 à 5 atomes d'halogène choisis parmi le fluor, le chlore, le brome et l'iode, ou par 1 à 5 groupes hydroxy, alcoxy en C₁₋₅, carboxyalkyle en C₁₋₅ ou cyano, un groupe alcényle en C₂₋₅, un groupe benzyle, un atome d'halogène choisi parmi le fluor, le chlore, le brome et l'iode, un groupe hydroxy, un groupe alcoxy en C₁₋₅, un groupe amino, un groupe (monoxyalkyle en C₁₋₅)-amino, un groupe di(alkyle en C₁₋₅)amino, un groupe (cycloalkyle en C₃₋₁₀)amino, un groupe pipéridino, pipérazino ou morpholino, un groupe carboxyle, un groupe carboxy(alkyle en C₁₋₅), un groupe carboxy(alcényle en C₂₋₅), un groupe carboxyamide, un groupe N-(alkyle en C₁₋₅)-carboxyamide, un groupe N,N-di(alkyle en C₁₋₅)carboxyamide, un groupe cyano, un groupe nitro, un groupe sulfonique, un groupe (alkyle en C₁₋₅)sulfonique, un groupe arylsulfonique choisi parmi les groupes benzènesulfonique, p-toluènesulfonique et p-chlorotoluènesulfonique, ou un groupe aryle choisi parmi les résidus phényle, diphényle et naphtyle,
R⁵ et R⁶ représentent tous les deux un groupe méthyle,
A représente un groupe arène monocyclique ou polycyclique contenant au moins une fonction carbonyle (C=O) sur le noyau ou dans la chaîne latérale, et qui est choisi parmi ceux représentés par les formules (II), (III), (IV), (V) ou (VI) suivantes : 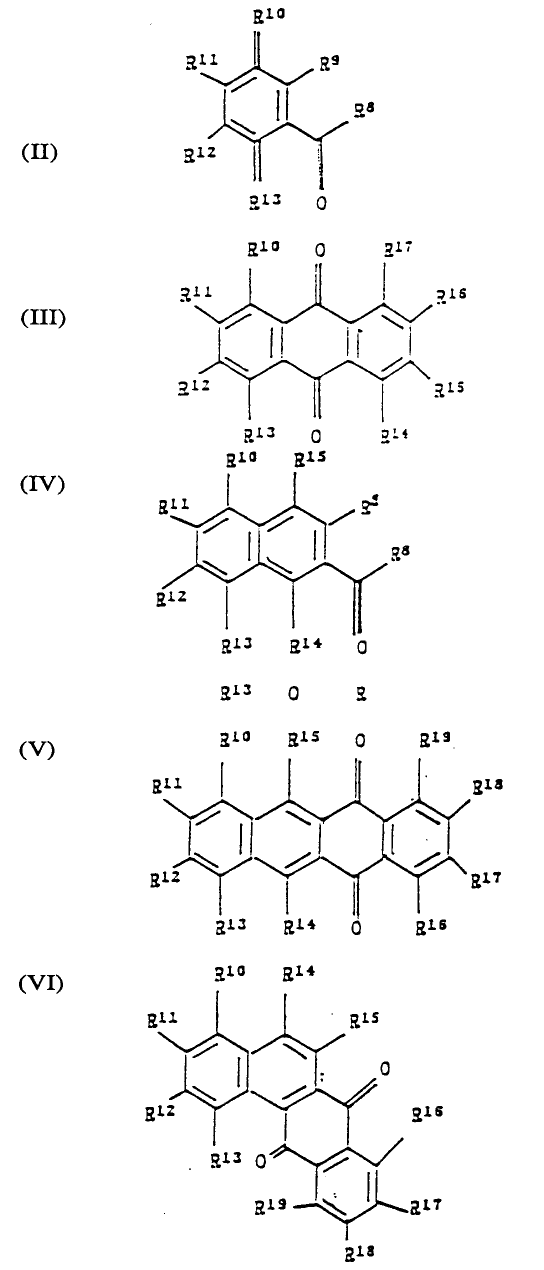 dans lesquelles
R⁸ représente un atome d'hydrogène, un atome d'halogène choisi parmi le chlore et le brome, un groupe alkyle en C₁₋₅ à chaîne linéaire ou ramifiée, un groupe alcényle en C₂₋₅, un groupe aryle monocyclique ou polycyclique, ou un groupe hétéroaryle choisi parmi les résidus phényle, naphtyle, anthracyle, furanyle, thiophényle, quinolyle et pyrrolyle, ou un tel groupe aryle ou hétéroaryle substitué par 1 à 4 groupes choisis parmi les atomes d'halogène tel que le fluor, le chlore et le brome, les groupes alkyle en C₁₋₅ à chaîne linéaire ou ramifiée, hydroxy, alcoxy en C₁₋₅, nitro, cyano, amino, mono(alkyle en C₁₋₅)amino, di(alkyle en C₁₋₅)amino, pipéridino, pipérazino ou morpholino,
deux résidus adjacents parmi les résidus R¹⁰ à R¹³ représentent les positions de fusion avec le noyau oxazine dans la formule générale (I), les autres ayant la même signification que R⁹ et R¹⁴ à R¹⁹,
R⁹ et R¹⁴ à R¹⁹ représentent un atome d'hydrogène, un groupe alkyle en C₁₋₅ à chaîne linéaire ou ramifiée, un groupe alkyle en C₁₋₅ substitué par 1 à 5 atomes d'halogène choisis parmi le fluor, le chlore, le brome et l'iode, par 1 à 5 groupes hydroxy, alcoxy en C₁₋₅, carboxy(alkyle en C₁₋₅) ou cyano, un groupe alcényle en C₂₋₅, un groupe benzyle, un atome d'halogène choisi parmi le fluor, le chlore, le brome et l'iode, un groupe hydroxy, un groupe alcoxy en C₁₋₅, un groupe carboxyle, un groupe carboxy(alkyle en C₁₋₅), un groupe carboxy(alcényle en C₂₋₅), un groupe carboxyamide, un groupe N-(alkyle en C₁₋₅)-carboxyamide, un groupe N,N-di(alkyle en C₁₋₅)carboxyamide, un groupe cyano, un groupe nitro, un groupe sulfonique, un groupe (alkyle en C₁₋₅)sulfonique, un groupe arylsulfonique choisi parmi les groupes benzènesulfonique, p-toluènesulfonique et p-chlorotoluènesulfonique, ou un groupe aryle choisi parmi les résidus phényle, diphényle et naphtyle,
ledit procédé comprenant l'étape consistant à faire réagir un dérivé nitroso des composés de formule (II), (III), (IV), (V) ou (VI) dans lesquelles un de deux résidus adjacents des groupes R¹⁰ à R¹³ représente un groupe -N=O et l'autre un groupe -OR²⁰, où R²⁰ représente un atome d'hydrogène, un cation métallique choisi parmi le sodium, le potassium le lithium et le cuivre, un cation ammonium ou di(alkyle en C₁₋₅)ammonium, un groupe (alkyle en C₁₋₅)carbonyle, un groupe (alkyle en C₁₋₅)sulfonyle ou un groupe arylsulfonyle,
avec un composé de formule (VII) 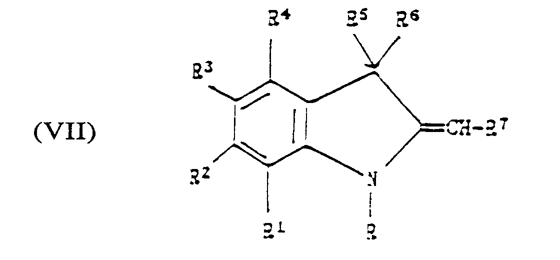 dans laquelle les substituants R et R¹ à R⁷ ont la signification indiquée ci-avant.

2. Procédé conforme à la revendication 1, caractérisé en ce que dans la formule (I)
R représente un groupe méthyle,
R¹ à R⁴, identiques ou différents, représentent chacun indépendamment un atome d'hydrogène, un atome de fluor, de chlore ou de brome, ou un groupe méthyle, isopropyle, trifluorométhyle, hydroxyméthyle, benzyle, hydroxy, méthoxy, amino, pipéridino, morpholino, carboxyle, carboxyméthyle, N,N-diméthylcarboxyamide, cyano, nitro ou phényle,
R⁵ et R⁶ représentent tous les deux un groupe méthyle,
R⁷ représente un atome d'hydrogène,
A correspond à un des groupes de formule (II) à (VI) dans lesquelles R⁸ représente un atome d'hydrogène, ou un groupe méthyle, isopropyle, phényle, p-N,N-diméthylaminophényle, p-cyanophényle, p-nitrophényle, p-méthoxyphényle, naphtyle, 2-thiophényle, 2-furanyle ou 4-pyridyle,
deux résidus adjacents parmi les résidus R¹⁰ à R¹³ représentent les positions de fusion avec le noyau oxazine dans la formule générale (I), les autres représentant indépendamment un atome d'hydrogène, un atome de fluor, de chlore ou de brome, ou un groupe méthyle, isopropyle, trifluorométhyle, hydroxyméthyle, benzyle, hydroxy, carboxy, carboxyméthyle, N,N-diméthylcarboxyamide, cyano, nitro ou phényle,
R⁹ et R¹⁴⁻¹⁹ représentent chacun indépendamment un atome d'hydrogène, un atome de fluor, de chlore ou de brome, ou un groupe méthyle, isopropyle, trifluorométhyle, hydroxyméthyle, benzyle, hydroxy, méthoxy, carboxyle, carboxyméthyle, N,N-diméthylcarboxyamide, cyano, nitro ou phényle.

3. Procédé conforme à la revendication 1, caractérisé en ce que les composés photochromes et thermochromes sont
la 1,3,3-triméthyl-8'-oxophényl-spiro-indoline-2,3'-[3H]naphto(2,1-b)-1,4-oxazine, et
la 1,1,1-triméthyl-spiro-indoline-2,3'-[3H]benzo[a]anthracène-7',12'-dione(3,4-b)-1,4-oxazine.

4. Procédé conforme à la revendication 1, caractérisé en ce que le composé (VII) est ajouté à une solution ou suspension du dérivé nitroso d'un des composés de formule (II), (III), (IV), (V) ou (VI), dans un solvant organique inerte, éventuellement en présence d'une amine tertiaire, en opérant à une température comprise entre 0 et 150 °C et de préférence entre 0 et 80°C, pendant une période comprise entre environ 1 minute et 24 heures.

5. Procédé pour préparer des articles en un matériau polymère utilisés comme filtres solaires, articles optiques, mémoires optiques, imprimés, photographies, tissus, articles décoratifs et jouets, caractérisés en ce qu'ils contiennent au moins un composé photochrome préparé conformément aux revendications 1 à 3.
